# EUROPEAN PATENT APPLICATION

(11) **EP 4 099 009 A1**
(43) Date of publication of application: **07.12.2022**
(21) Application number: 20916800.4
(22) Date of filing: 30.01.2020
(51) Int. Cl.: G01N 29/265

(54) **NON-DESTRUCTIVE INSPECTION DEVICE AND NON-DESTRUCTIVE INSPECTION METHOD**

(71) Applicant: Mitsubishi Heavy Industries, Ltd., 100-8332 Tokyo (JP)
(72) Inventor: SHIBUTANI, Takashi, Tokyo 100-8332 (JP); HASEGAWA, Osamu, Tokyo 100-8332 (JP)
(74) Representative: Hoffmann Eitle
(86) International application number: PCT/JP2020/003306
(87) International publication number: WO 2021/152756

(57) **Abstract**

This non-destructive inspection device comprises an inspection element having an inspection probe and a signal cable connected to the inspection probe and a moving element for moving the inspection element. The moving element comprises a carrier that supports the inspection element and moves along the movement path of the inspection element and a carrier guide that guides the carrier along the movement path. The carrier is configured through the linking of a plurality of covers that are more rigid than the signal cable. The carrier guide preferably comprises a first path that is linear, a second path that turns back and has one end that is continuous with the first path, and a third path that is linear and is continuous with the other end of the second path.

## Description

### Technical Field

The present disclosure relates to a device for non-destructive inspection of a long inspection target.

### Background Art

A composite component of an aircraft or the like is manufactured by laminating resins each containing carbon fiber and heat-curing the resins. In a case where interlayer delamination occurs due to a foreign substance that is mixed with the resins when the resins are laminated or due to insufficient pressure joining, there may be a shortage of strength. Therefore, inspection for a foreign substance and interlayer delamination is performed by means of ultrasonic flaw detection, for example. To find the position of a foreign substance through ultrasonic flaw detection, it is necessary to accurately find the position of an inspection probe in an inspection target. Generally, a signal cable for transmission or the like of a signal related to inspection is connected to the inspection probe.

In the case of a tubular inspection target, an inspection probe is supposed to be inserted through an opening at one end of the inspection target. However, in a case where the inspection target is long, a signal cable for the probe is also long and a weight including the weight of the signal cable is large. In this case, for example, it is possible to insert the inspection probe into a deep part of the inspection target by feeding the signal cable. However, since the rigidity of the signal cable is low, the signal cable may buckle on the way and become not able to be inserted further.

With regard to such a problem, proposed in PTL 1 is a method of connecting an internal probe and an external driving source to each other by means of a magnetic coupling and driving the internal probe and the external driving source. According to the method proposed in PTL 1, it is easy to drive an inspection probe. However, in a case where the thickness of a tubular inspection target is large, there is a decrease in adsorption force of the magnetic coupling. Therefore, transmission of power may become impossible due to the magnetic coupling falling off while the inspection probe is running or accurate detection of the position of the inspection probe may become impossible due to occurrence of positional deviation of the driving source and the inspection probe.

### Citation List

### Patent Literature

[PTL 1] Japanese Unexamined Patent Application Publication No. 2008-528971

### Summary of Invention

### Technical Problem

Therefore, the present disclosure provides a device with which it is possible to perform high-accuracy non-destructive inspection such as ultrasonic flaw detection inside an inspection target even in a case where the inspection target is long and tubular.

### Solution to Problem

According to an aspect of the present disclosure, there is provided a non-destructive inspection device including an inspection element that includes an inspection probe and a signal cable connected to the inspection probe and a moving element that moves the inspection element while supporting the inspection element.

The moving element according to the aspect of the present disclosure includes a carrier that moves along a movement path of the inspection element and a carrier guide that guides the carrier along the movement path.

The carrier according to the aspect of the present disclosure is configured by connecting a plurality of covers that have a rigidity higher than a rigidity of the signal cable.

According to another aspect of the present disclosure, there is provided a non-destructive inspection method for a non-destructive inspection device including an inspection element that includes an inspection probe and a signal cable connected to the inspection probe and a moving element that moves the inspection element while supporting the inspection element.

The moving element includes a carrier that supports the inspection element and moves along a movement path of the inspection element and a carrier guide that guides the carrier along the movement path. The carrier is configured by connecting a plurality of covers that have a rigidity higher than a rigidity of the signal cable.

The inspection method according to the other aspect of the present disclosure includes a step (a) of moving the inspection element along an inspection target by means of the moving element and a step (b) of non-destructively inspecting the inspection target by causing the inspection probe to function during movement of the inspection element.

### Advantageous Effects of Invention

In the case of the non-destructive inspection device and the non-destructive inspection method according to the aspects of the present disclosure, the carrier is configured by connecting the plurality of covers that have the rigidity higher than the rigidity of the signal cable. Therefore, it is possible to perform high-accuracy non-destructive inspection inside an inspection target even in a case where the inspection target is long and tubular.

### Brief Description of Drawings

Fig. 1 is a side view showing a non-destructive inspection device according to the present embodiment.
Fig. 2 is a side view showing the non-destructive inspection device according to the present embodiment with an inspection target installed therein.
Fig. 3 is a view showing an example of the way in which a cover according to the present embodiment supports a signal cable.
Fig. 4 is a plan view (PV) and a side view (SV) showing the cover.
Fig. 5 is a view that shows a procedure for inspection of a tubular inspection target that is performed by using a non-destructive inspection device 1 in Fig. 1.
Fig. 6 is a view subsequent to Fig. 2 that shows the procedure for the inspection of the tubular inspection target that is performed by using the non-destructive inspection device in Fig. 1.
Fig. 7 is a view showing a preferable embodiment of the non-destructive inspection device 1 of Fig. 1.
Fig. 8 is a view showing a preferable embodiment of the non-destructive inspection device 1 of Fig. 1. Description of Embodiments

Hereinafter, a non-destructive inspection device 1 according to an embodiment will be described with reference to the drawings.

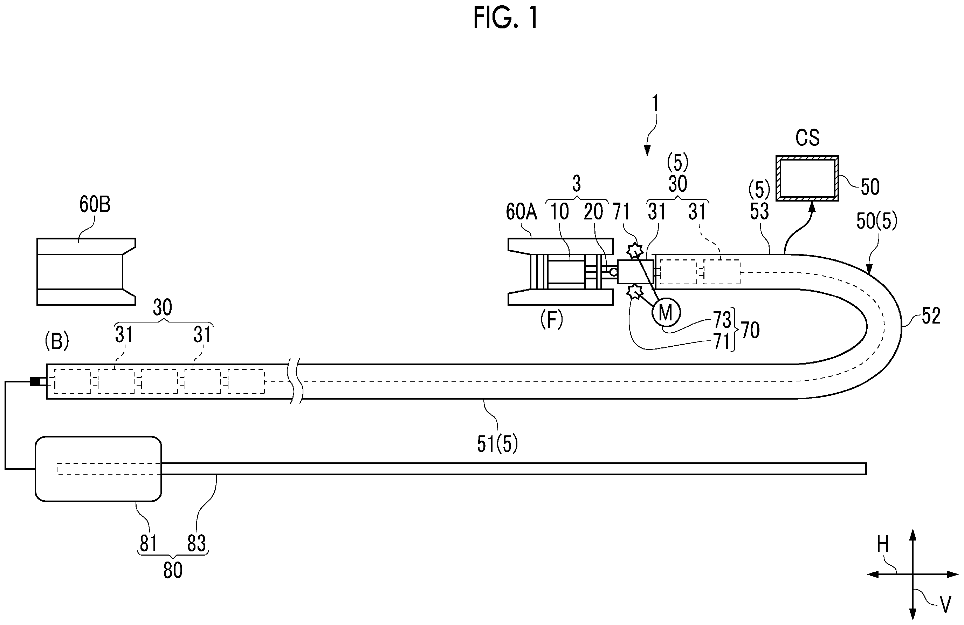

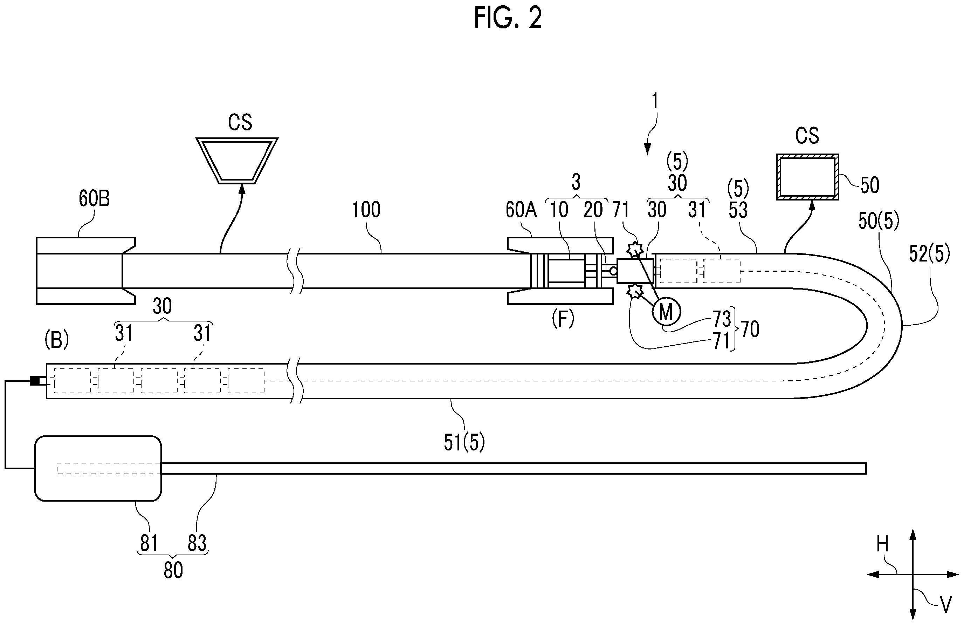

As shown in Fig. 2, the non-destructive inspection device 1 inspects, by means of a non-destructive test (JIS Z 2300 (0126)) with ultrasonic flaw detection, whether or not there is a flaw in a long inspection target 100 that has a trapezoidal or rectangular shape with a hollow cross section (CS), for example. Note that in the non-destructive inspection device 1, a vertical direction V and a horizontal direction H are defined as shown in Figs. 1 and 2.

### [Overall Configuration]

As shown in FIGS. 1 and 2, the non-destructive inspection device 1 includes an inspection element 3 including an inspection probe 10 and a signal cable 20 connected to the inspection probe 10. In addition, the non-destructive inspection device 1 includes a carrier 30 as a moving element 5 that covers the periphery of the signal cable 20. In addition, the non-destructive inspection device 1 includes a carrier guide 50 that guides the movement path of a plurality of the carriers 30 accommodating the signal cable 20. In addition, the non-destructive inspection device 1 includes a first support 60A that supports the inspection probe 10 inserted into the inspection target 100 and a second support 60B that supports the inspection probe 10 that has passed through the inspection target 100. Furthermore, the non-destructive inspection device 1 includes a movement mechanism 70 that moves the carrier 30 forward and backward and a control mechanism 80 that controls the operation of the non-destructive inspection device 1.

The non-destructive inspection device 1 inspects the inspection target 100 with the inspection probe 10, the signal cable 20, and the carrier 30 being moved together along the carrier guide 50, the inspection probe 10, the signal cable 20, and the carrier 30 being moved by the movement mechanism 70 serving as a driving source. Note that, regarding the non-destructive inspection device 1, as shown in Figs. 1 and 2, a side on which the inspection probe 10 is provided will be referred to as a front side (F) and a side opposite thereto will be referred to as a rear side (B). However, the above-described definition is relative.

### [Inspection Probe 10]

The non-destructive inspection device 1 inspects the inspection target 100 by means of, for example, an ultrasonic test (UT).

Here, since ultrasonic waves have a short wavelength, have a property of proceeding straight, and are easily reflected at an interface between a solid and a liquid or gas, the ultrasonic waves are used for detection of a flaw in a material. Regarding an ultrasonic flaw detection method, a method called a pulse reflection method is mainly used in which a voltage generated by a pulse oscillator is applied to a vibrator to generate an ultrasonic wave pulse having a frequency of 500 kHz to 10 MHz.

Generally, the inspection probe 10 is combined with a vibrator and a damper and is incorporated in a housing. The vibrator is formed of a piezoelectric material selected from quartz, barium titanate, lead zirconate titanate, lithium sulfate, and the like.

The inspection target 100 is formed of any material such as metal or a composite material. Examples of the composite material include fiber reinforced plastic (FRP). Examples of the fiber reinforced plastic include glass fiber reinforced plastic (GFRP) and carbon fiber reinforced plastic (CFRP). The GFRP is obtained by hardening glass fiber with polyester resin, vinyl ester resin, epoxy resin, phenol resin, or the like and the CFRP is obtained by hardening carbon fiber with the same resin as above.

In the present embodiment, the non-destructive inspection device 1 based on ultrasonic flaw detection is used as an example. However, the present disclosure can be applied to other types of non-destructive tests. That is, the present disclosure can be applied to a radiographic test (RT), a magnetic test (MT), and an eddy current test (ET) in which an inspection probe is movable and a signal cable is provided.

### [Signal Cable 20]

The signal cable 20 transmits a voltage applied to the vibrator of the inspection probe 10 and transmits the reflected wave of an ultrasonic wave received by the vibrator to the control mechanism 80. The signal cable 20 includes a core wire that is formed of a highly conductive material such as a copper alloy and a covering layer that is formed of an electrically insulating material such as a resin and covers the periphery of the core wire.

The signal cable 20 has a low rigidity. Therefore, in a case where the long signal cable 20 is fed, the weight of the inspection probe 10 is added to the weight of the signal cable 20 and thus the signal cable 20 buckles. Therefore, for the non-destructive inspection device 1, the carrier 30 is used in order to prevent the signal cable 20 from buckling.

### [Carrier 30]

The carrier 30 has a higher rigidity than the signal cable 20, and moves the inspection probe 10 and the signal cable 20 forward and backward while supporting the signal cable 20. As shown in Figs. 1 and 2, the carrier 30 is formed by connecting a plurality of covers 31. Note that, in Figs. 1 and 2, the covers 31 between the front side (F) and the rear side (B) are not shown.

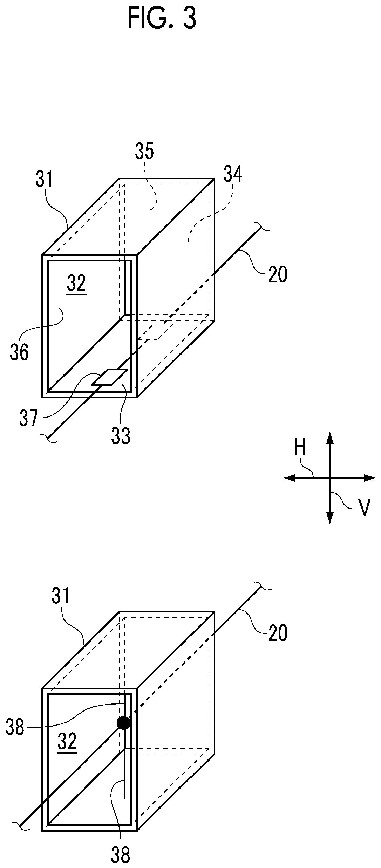

As shown in Fig. 3, each of the covers 31 is a tubular member of which an opening has a rectangular shape and supports the signal cable 20 with the signal cable 20 accommodated in an internal space 32 thereof. Note that the signal cable 20 may be supported by all of the covers 31 constituting the carrier 30 or may be supported intermittently by being supported by every fourth cover 31. In any case, the signal cable 20 is supported by the plurality of covers 31 and thus the signal cable 20 can be reliably supported by the carrier 30.

The covers 31 are preferably formed of a metallic material or a resin material. The covers 31 formed of a metallic material have a high durability and the covers 31 made of a resin material are lightweight.

The signal cable 20 may be supported by the covers 31 in any manner. For example, as shown in the upper part of Fig. 3, an adhesive tape 37 or the like may be attached to an inner peripheral surface of any of four side walls 33, 34, 35, and 36 constituting the cover 31. In addition, as shown in the lower part of Fig. 3, a support 38 that supports the signal cable 20 may be provided inside the cover 31 over the vertical direction V and the signal cable 20 may be fastened to the support 38.

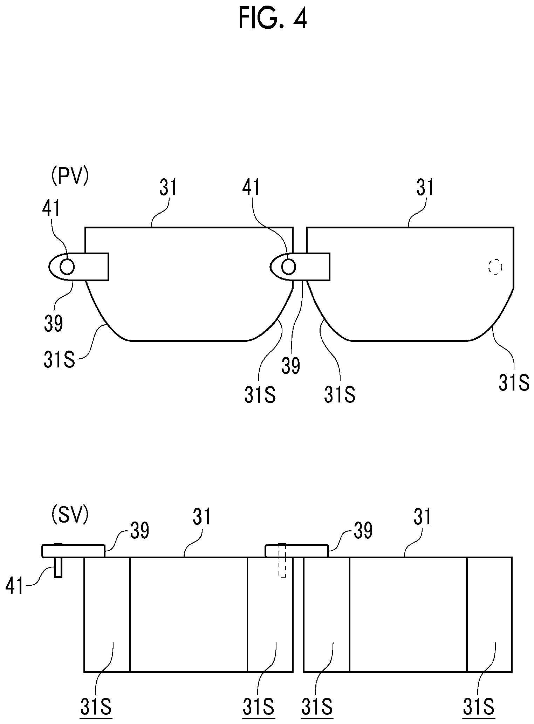

As shown in Fig. 4, front and rear end portions of the covers 31 that move forward and backward are provided with hooks 39. The hook 39 of one of preceding and following covers 31 is connected to the other of the preceding and following covers 31 by means of a pin 41. Accordingly, the carrier 30 composed of the plurality of covers 31 connected in a row moves forward and backward together with the signal cable 20. During the forward movement and the backward movement, each cover 31 receives a mechanical load from preceding and following covers 31 but has such a rigidity that the load does not cause deformation. Note that, in the drawing, "PV" means a plan view and "SV" means a side view.

A front end and a rear end of each of the covers 31 shown in Fig. 4 are provided with curved surfaces 31S so that a mechanical load acting when the covers 31 come into contact with each other while being curved as the carrier 30 is reduced.

### [Carrier Guide 50]

Next, the carrier guide 50 will be described.

The carrier guide 50 guides the carrier 30 to move forward and backward along a predetermined movement path. As shown in Figs. 1 and 2, the carrier guide 50 according to the present embodiment includes a first path 51 that is provided on a lower side in the vertical direction V and is linear, a second path 52 that is connected to one end of the first path 51 and is arc-shaped, and a third path 53 that is connected to one end of the second path 52, is provided on an upper side in the vertical direction V, and is linear. As described above, the carrier guide 50 is folded at the second path 52 to form a U-like shape. In addition, the carrier guide 50 is formed in two stages with the first path 51 and the third path 53 being on the upper and lower sides in the vertical direction V.

Since the movement path of the carrier guide 50 is folded in this manner, a long movement path can be secured in a narrow space. As described here, the first path 51 and the third path 53 can be arranged in two stages in the horizontal direction H instead of being arranged in two stages in the vertical direction V. It is possible to reduce the size of a required space in the water level direction H when arranging the paths in two stages in the vertical direction V and it is possible to reduce the size of a required space in the vertical direction V when arranging the paths in two stages in the horizontal direction H. Therefore, in a case where the movement path is to be folded, a direction in which the movement path is folded back may be set in accordance with the surrounding environment.

The outer shape of a cross section (CS) of the carrier guide 50 is rectangular and tubular throughout an area from the first path 51 to the third path 53. The carrier 30 moves forward and backward along the movement path composed of a space closed by the carrier guide 50. Regarding the space, it is preferable that gaps around the covers 31 are minimized on the assumption that the carrier 30 can move forward and backward. Since each cover 31 moves forward and backward without meandering, the amount of movement of the inspection probe 10 can be specified with high accuracy.

### [First Support 60A and Second Support 60B]

As shown in Fig. 2, the first support 60A and the second support 60B support the inspection target 100 at both of end portions thereof. An ultrasonic test is performed by moving the inspection probe 10 forward and backward with the inspection target 100 supported by the first support 60A and the second support 60B.

### [Movement Mechanism 70]

The movement mechanism 70 is provided at a tip end of the third path 53 and at a position corresponding to the foremost cover 31 of the carrier 30 in a standby state. When inspection is started, the movement mechanism 70 comes into contact with the covers 31 and applies thrust in a forward movement direction in this order: the foremost cover 31, the second foremost cover 31, the third foremost cover 31, ... and so forth. Since the covers 31 have a high rigidity, by applying thrust to any of the covers 31, it is possible to move the cover 31 preceding the cover 31 to which the thrust is applied and the cover 31 following the cover 31 to which the thrust is applied. When the foremost cover 31 reaches a terminal end of the inspection target 100, thrust is applied to the covers 31 sequentially in a backward movement direction. The thrust in the backward movement direction is applied until the foremost cover 31 exits the inspection target 100 and reaches the first support 60A.

The movement mechanism 70 in the present embodiment includes a pair of thrust rollers 71 pressed against the side walls 33 to 36 of at least one of the covers 31 and an electric motor 73 driving the thrust rollers 71. It is preferable that the electric motor 73 can perform rotation in forward and reverse directions and can change the rotation speed steplessly.

### [Control Mechanism 80]

The control mechanism 80 includes an inspection machine 81 that supplies a voltage to the inspection probe 10 via the signal cable 20 and receives an electric signal based on a reflected wave received by the inspection probe 10 and a guide 83 that guides movement of the inspection machine 81. The inspection machine 81 moves forward and backward while being guided by the guide 83 as the inspection probe 10 and the carrier 30 move.

### [Inspection Procedure]

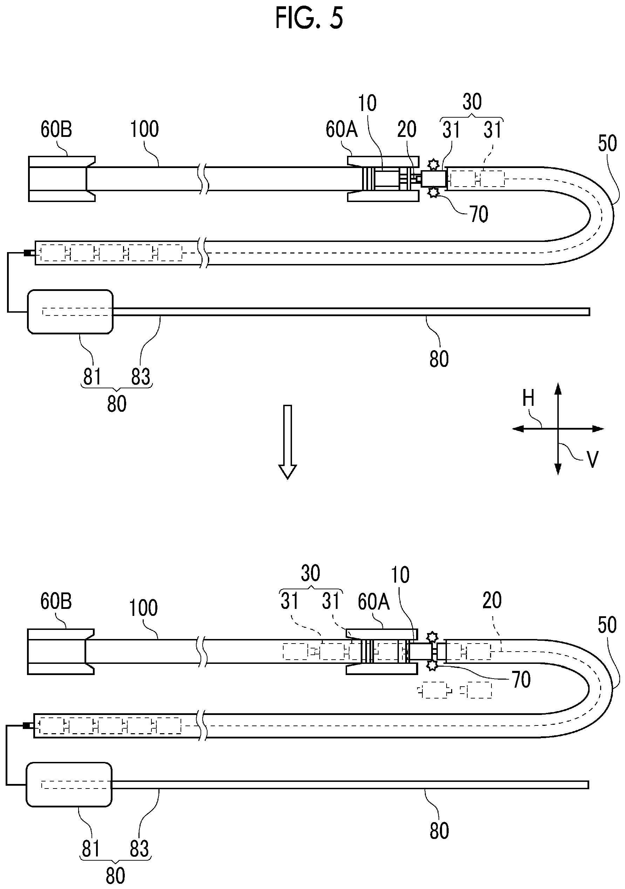

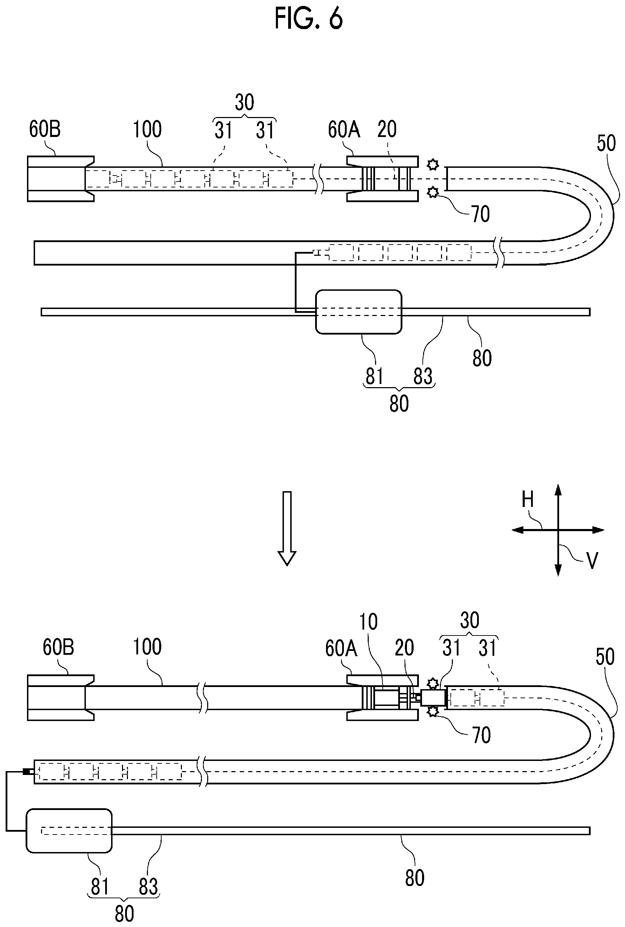

Next, with reference to Figs. 5 and 6, a procedure for performing an ultrasonic test on the inspection target 100 using the non-destructive inspection device 1 will be described. First, the non-destructive inspection device 1 moves the inspection probe 10 forward to feed the inspection probe 10 to the tip end of the inspection target 100 and performs ultrasonic flaw detection by receiving a reflected wave while causing the vibrator to perform ultrasonic wave infiltration during backward movement of the inspection probe 10.

As shown in the upper part of Fig. 5, the inspection target 100 is brought in and supported by the first support 60A and the second support 60B when the inspection probe 10, the signal cable 20, and the carrier 30 are at standby positions thereof. In this manner, the ultrasonic test becomes ready to be carried out.

As shown in the lower part of Fig. 5, the movement mechanism 70 is driven such that the inspection probe 10, the signal cable 20, and the carrier 30 are moved forward. As shown in the upper part of Fig. 6, when the inspection probe 10 moves forward and reaches the tip end of the inspection target 100, application of thrust performed by the movement mechanism 70 is temporarily stopped.

Then, as shown in the lower part of Fig. 6, thrust in a reverse direction is applied to the covers 31 from the movement mechanism 70 so that the inspection probe 10, the signal cable 20, and the carrier 30 are moved backward. During backward movement of the inspection probe 10, the vibrator of the inspection probe 10 is vibrated such that an ultrasonic wave is emitted toward the inspection target 100 and a reflected wave from the inspection target 100 is received for flaw detection. Although not shown, when the inspection probe 10, the signal cable 20, and the carrier 30 return to the standby positions, the application of thrust performed by the movement mechanism 70 is stopped. In this manner, a series of ultrasonic flaw detection is finished.

Since the covers 31 have a high rigidity, the entire length of the carrier 30 does not change during movement of the signal cable 20 and the carrier 30 as described above and there is only a slight change even if there is a change in entire length of the carrier 30. Therefore, the position accuracy of the inspection probe 10 provided at a front end of the carrier 30 can be secured.

### [Effect]

As described above, in the case of the non-destructive inspection device 1, the inspection probe 10 and the signal cable 20 can be moved forward or backward reliably with the carrier 30 being moved forward or backward. Furthermore, since the inspection probe 10 and the signal cable 20 are supported by the carrier 30, it is possible to prevent deformation such as bending or buckling of the signal cable 20. Therefore, according to the non-destructive inspection device 1, the amount of movement of the inspection probe 10 can be specified with high accuracy and thus high-accuracy ultrasonic flaw detection can be performed even in a case where the inspection target 100 is long and tubular.

### [Second Embodiment]

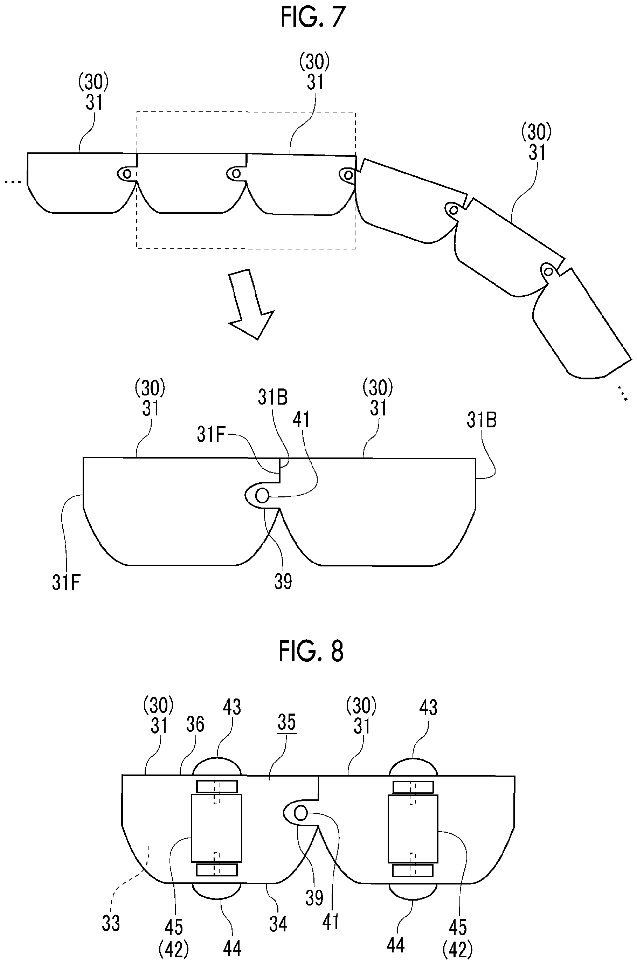

Next, a second embodiment of the present disclosure will be described with reference to Fig. 7.

The second embodiment is for specifying the positional relationship between the adjacent covers 31. More specifically, the preceding cover 31 and the following cover 31 are brought into surface-contact with each other. Therefore, in addition to the presence of flat surfaces at a rear surface 31B of the preceding cover 31 and a front surface 31F of the following cover 31, positions at which the hook 39 and the pin 41 for connection between the preceding cover 31 and the following cover 31 are adjusted.

Since preceding and following covers 31 come into surface-contact with each other at the rear surface 31B and the front surface 31F, a pressure applied to each cover 31 can be reduced and the rigidity of the covers 31 can be improved. Accordingly, it is possible to reduce the degree of deformation of each cover 31 or to prevent deformation of each cover 31. Therefore, it is possible to move the inspection probe 10 and the signal cable 20 reliably and accurately via the covers 31. It is possible to prevent an error in feeding amount measurement with respect to the inspection probe 10 and the signal cable 20 caused by looseness of the covers 31 connected to each other and it is possible to improve the accuracy of position detection of the inspection probe 10.

### [Third Embodiment]

Next, a third embodiment of the present disclosure will be described with reference to Fig. 8.

The gist of the third embodiment is to provide a friction reduction configuration for the side walls 34 to 36 of the covers 31. Specifically, as shown in Fig. 8, rollers 42, 43, 44, and 45 rotatably provided on the respective side walls 33, 34, 35, and 36 are provided. Each of the rollers 42 to 44 can rotate around an axis thereof when resistance is applied thereto.

In the case of the covers 31 including the rollers 42 to 45, the rollers 42 to 45 rotate during movement in the carrier guide 50 and movement in the inspection target 100 although the rollers 42 to 45 come into contact with the carrier guide 50 and the inspection target 100 during the movement. Therefore, friction can be reduced. Therefore, at the time of flaw detection, it is possible to prevent thrust applied by the movement mechanism 70 from being insufficient with respect to frictional resistance from the carrier guide 50 and the inspection target 100 that is caused by the weights of the signal cable 20 and the carrier 30. In addition, it is possible to reduce wear of the covers 31, the carrier guide 50, and the inspection target 100 caused by contact between the covers 31 and the carrier guide 50 and contact between the covers 31 and the inspection target 100.

The rollers 42 to 45 are merely an example of friction reducing means. A spherical rotating body can be used as the friction reducing means. In addition to the rotating body, a member having a low coefficient of friction may be provided as the friction reducing means instead of the rollers 42 to 45. Examples of the material having a low coefficient of friction include polytetrafluoroethylene (PTFE) and the like.

### [Appendix]

The non-destructive inspection device 1 and the inspection method described in the above embodiment are understood as follows.

### [Effect of Non-Destructive Inspection Device 1 According To First Aspect]

The non-destructive inspection device 1 according to a first aspect includes the inspection element 3 that includes the inspection probe 10 and the signal cable 20 connected to the inspection probe 10 and the moving element 5 that moves the inspection element 3.

The moving element 5 includes the carrier 30 that supports the inspection element 3 and moves along the movement path of the inspection element 3 and the carrier guide 50 that guides the carrier 30 along the movement path.

The carrier 30 is configured by connecting the plurality of covers 31 that have a rigidity higher than a rigidity of the signal cable 20.

### [Effect of Non-Destructive Inspection Device 1 According To First Aspect]

According to the non-destructive inspection device 1 of the first aspect, the inspection probe 10 and the signal cable 20 can be moved forward or backward reliably with the carrier 30 being moved forward or backward. Furthermore, since the inspection probe 10 and the signal cable 20 are supported by the carrier 30, it is possible to prevent deformation such as bending or buckling of the signal cable 20. Therefore, according to the non-destructive inspection device 1, the amount of movement of the inspection probe 10 can be specified with high accuracy and thus high-accuracy ultrasonic flaw detection can be performed even in a case where the inspection target 100 is long and tubular.

### [Configuration of Non-Destructive Inspection Device 1 According To Second Aspect]

In the non-destructive inspection device 1 according to a second aspect, the carrier guide 50 includes the first path 51 that is linear, the second path 52 of which one end is connected to the first path 51 and that serves as a folded path, and the third path 53 that is connected to the other end of the second path 52.

### [Effect of Non-Destructive Inspection Device 1 According To Second Aspect]

According to the non-destructive inspection device 1 of the second aspect, since the movement path of the carrier guide 50 is folded, a long movement path can be secured in a narrow space.

### [Configuration of Non-Destructive Inspection Device 1 According To Third Aspect]

In the non-destructive inspection device 1 according to the third aspect, the first path 51 and the third path 53 are arranged in the vertical direction V or arranged in the horizontal direction H.

### [Effect of Non-Destructive Inspection Device 1 According To Third Aspect]

According to the non-destructive inspection device 1 of the third aspect, it is possible to reduce the size of a required space in the water level direction H when arranging the paths in two stages in the vertical direction V and it is possible to reduce the size of a required space in the vertical direction V when arranging the paths in two stages in the horizontal direction H. Therefore, in a case where the movement path is to be folded, a direction in which the movement path is folded back may be set in accordance with the surrounding environment.

### [Configuration of Non-Destructive Inspection Device 1 According To Fourth Aspect]

In the non-destructive inspection device 1 according to a fourth aspect, the carrier guide 50 is composed of a tubular body in which the hollow movement path is provided and the carrier 30 moves through the movement path in the tubular body.

### [Effect of Non-Destructive Inspection Device 1 According To Fourth Aspect]

According to the non-destructive inspection device 1 of the fourth aspect, in a case where gaps around the covers 31 are minimized on the assumption that the carrier 30 can move forward and backward, each cover 31 can move forward and backward without meandering. Accordingly, the amount of movement of the inspection probe 10 can be specified with high accuracy.

### [Configuration of Non-Destructive Inspection Device 1 According To Fifth Aspect]

In the non-destructive inspection device 1 according to a fifth aspect, a hollow cable accommodation region is provided in the cover 31 and the signal cable 20 is supported by the plurality of covers 31 in the cable accommodation regions.

### [Effect of Non-Destructive Inspection Device 1 According To Fifth Aspect]

According to the non-destructive inspection device 1 of the fifth aspect, the signal cable 20 is supported by the plurality of covers 31 and thus the signal cable 20 can be reliably supported by the carrier 30.

### [Configuration of Non-Destructive Inspection Device 1 According To Sixth Aspect]

The non-destructive inspection device 1 according to a sixth aspect further includes the movement mechanism 70 that comes into contact with the covers 31 and applies thrust to the covers 31.

### [Effect of Non-Destructive Inspection Device 1 According To Sixth Aspect]

According to the non-destructive inspection device 1 of the sixth aspect, since the covers 31 have a high rigidity, by applying thrust to any of the covers 31, it is possible to move the cover 31 preceding the cover 31 to which the thrust is applied and the cover 31 following the cover 31 to which the thrust is applied.

### [Configuration of Non-Destructive Inspection Device 1 According To Seventh Aspect]

In the non-destructive inspection device 1 according to a seventh aspect, the cover 31 includes a front surface and a rear surface that come into plane-contact with preceding and following covers 31.

### [Effect of Non-Destructive Inspection Device 1 According To Seventh Aspect]

According to the non-destructive inspection device 1 of the seventh aspect, since preceding and following covers 31 come into surface-contact with each other, a pressure applied to each cover 31 can be reduced and the rigidity of the covers 31 can be improved. Accordingly, it is possible to reduce the degree of deformation of each cover 31 or to prevent deformation of each cover 31. Therefore, it is possible to move the inspection probe 10 and the signal cable 20 reliably and accurately via the covers 31.

### [Configuration of Non-Destructive Inspection Device 1 According To Eighth Aspect]

In the non-destructive inspection device 1 according to an eighth aspect, at least one of the cover 31 and the carrier guide 50 includes a member that reduces friction with a counterpart.

### [Effect of Non-Destructive Inspection Device 1 According To Eighth Aspect]

According to the non-destructive inspection device 1 of the eighth aspect, it is possible to reduce wear of the covers 31, the carrier guide 50, and the inspection target 100.

### [Configuration of Non-Destructive Inspection Method According To First Aspect]

A non-destructive inspection method according to a first aspect is a non-destructive inspection method for the non-destructive inspection device 1 including the inspection element 3 that includes the inspection probe 10 and the signal cable 20 connected to the inspection probe 10 and the moving element 5 that moves the inspection element 3 while supporting the inspection element 3.

The moving element 5 includes the carrier 30 that supports the inspection element 3 and moves along the movement path of the inspection element 3 and the carrier guide 50 that guides the carrier 30 along the movement path.

The carrier 30 is configured by connecting the plurality of covers 31 that have a rigidity higher than a rigidity of the signal cable 20.

The inspection method according to the first aspect includes a step (a) of moving the inspection element 3 along the inspection target 100 by means of the moving element 5 and a step (b) of non-destructively inspecting the inspection target 100 by causing the inspection probe 10 to function during movement of the inspection element 3.

### [Effect of Non-Destructive Inspection Method According To First Aspect]

According to the non-destructive inspection method of the first aspect, the inspection probe 10 and the signal cable 20 can be moved forward or backward reliably with the carrier 30 being moved forward or backward. Furthermore, since the inspection probe 10 and the signal cable 20 are supported by the carrier 30, it is possible to prevent deformation such as bending or buckling of the signal cable 20. Therefore, according to the non-destructive inspection device 1, the amount of movement of the inspection probe 10 can be specified with high accuracy and thus high-accuracy ultrasonic flaw detection can be performed even in a case where the inspection target 100 is long and tubular.

In addition to the above, it is possible to select the configurations described in the above embodiments or change the configurations to other configurations as appropriate.

For example, regarding the movement mechanism 70, a rotary encoder and a linear encoder can be used as position detection means and the electric motor 73 can have a function as a rotary encoder. In addition, regarding the linear encoder, the covers 31 can have a sensor function and the carrier guide 50 can have a scale function. The rotary encoder and the linear encoder may be any of optical type encoders and magnetic type encoders.

In addition, as the movement mechanism 70, a combination of a slider attached to one end of each cover 31, a rack attached to the slider, a pinion gear meshing with the rack, and an electric motor rotationally driving the pinion gear can also be used. The driving of the slider is not limited to the above-described example and an electric linear motor, a ball screw, or the like can be used.

In addition, pulling by means of a rod or rope inserted from a side opposite to a probe insertion port may also be adopted.

### Reference Signs List

- 1: non-destructive inspection device
- 3: inspection element
- 5: moving element
- 10: inspection probe
- 20: signal cable
- 30: carrier
- 31: cover
- 50: carrier guide
- 51: first path
- 52: second path
- 53: third path
- 60A: first support
- 60B: second support
- 70: movement mechanism
- 71: thrust roller
- 73: electric motor

## Claims

1. A non-destructive inspection device comprising:
an inspection element that includes an inspection probe and a signal cable connected to the inspection probe; and
a moving element that moves the inspection element while supporting the inspection element,
wherein the moving element includes
a carrier that supports the inspection element and moves along a movement path of the inspection element, and
a carrier guide that guides the carrier along the movement path, and
the carrier is configured by connecting a plurality of covers that have a rigidity higher than a rigidity of the signal cable.

2. The non-destructive inspection device according to claim 1,
wherein the carrier guide includes a first path that is linear, a second path of which one end is connected to the first path and that serves as a folded path, and a third path that is connected to the other end of the second path and that is linear.

3. The non-destructive inspection device according to claim 2,
wherein the first path and the third path are arranged in a vertical direction or arranged in a horizontal direction.

4. The non-destructive inspection device according to any one of claims 1 to 3,
wherein the carrier guide is composed of a tubular body in which a movement path is provided, and
the carrier moves through the movement path in the tubular body.

5. The non-destructive inspection device according to any one of claims 1 to 4,
wherein a cable accommodation region is provided in the cover, and
the signal cable is supported by the plurality of covers in the cable accommodation regions.

6. The non-destructive inspection device according to any one of claims 1 to 5, further comprising:
a movement mechanism that comes into contact with the covers and applies thrust to the covers.

7. The non-destructive inspection device according to any one of claims 1 to 6,
wherein the cover includes a front surface and a rear surface that come into plane-contact with preceding and following covers.

8. The non-destructive inspection device according to any one of claims 1 to 7,
wherein at least one of the cover and the carrier guide includes a member that reduces friction with a counterpart.

9. A non-destructive inspection method for a non-destructive inspection device including an inspection element that includes an inspection probe and a signal cable connected to the inspection probe and a moving element that moves the inspection element while supporting the inspection element, the moving element including a carrier that supports the inspection element and moves along a movement path of the inspection element and a carrier guide that guides the carrier along the movement path, the carrier being configured by connecting a plurality of covers that have a rigidity higher than a rigidity of the signal cable, the method comprising:
a step (a) of moving the inspection element along an inspection target by means of the moving element; and
a step (b) of non-destructively inspecting the inspection target by causing the inspection probe to function during movement of the inspection element.
